# EUROPEAN PATENT APPLICATION

(11) **EP 1 002 875 A1**
(43) Date of publication of application: **24.05.2000**
(21) Application number: 98121830.8
(22) Date of filing: 17.11.1998
(51) Int. Cl.: C12Q 1/68, C07H 21/00, C12N 15/10

(54) **An oligonucleotide inhibitor of thermostable polymerase**

(71) Applicant: Mira Diagnostica GmbH, 51377 Leverkusen (DE)
(72) Inventor: Lunin, Vladimir Glebovich, 123098 Moskva (RU); Alexseev, Jakov Igorevich, 121596 Moskva (RU); Anisimova, Sveltana Sergeevna, 107113 Moskva (RU); Zavriev, Sergei Kiriakovich, 125422 Moskva (RU); Leiser, Robert-Matthias, 42697 Solingen (DE); Plobner, Lutz, 40699 Erkrath (DE)
(74) Representative: Meyers, Hans-Wilhelm, Dr.Dipl.-Chem.

(57) **Abstract**

An oligonucleotide inhibitor of 5'-3'exonuclease activity of thermostable polymerases, the oligonucleotide having a first structural domain (stem I), a second structural domain (loop), and a third structural domain (stem II), wherein
the third structural domain (stem II) is consisting of oligonucleotides having a nucleotide sequence which is complementary to the oligonucleotide sequence of the first structural domain (stem I), the first and third structural domain are forming a first double stranded structural element (stem) by hybridization of the complementary oligonucleotide sequences at ambient temperature,
the second structural domain (loop) comprising a structural element NN'XY, wherein
   - N and N' is a nucleotide residue of the pyrimidine type or
   - N is a nucleotide residue of the purine type and N' is a nucleotide residue of the pyrimidine type,
   - X is a structural element of nucleotide residues within the loop formed by a turn portion in the loop and a double stranded portion, wherein the double stranded portion is consisting of two to five nucleotide residues of any kind, and
   - the turn portion is consisting of one to four nucleotide residues of any kind,
   - Y is a structural element consisting of up to 4 nucleotide nucleotides of any kind.

## Description

The present invention concerns an oligonucleotide inhibitor of a thermostable polymerase, and a method of inhibiting a thermostable polymerase by using an oligonucleotide inhibitor of the present invention.

WO-A-96/41010 discloses a method for systematic evolution of ligands by exponential enrichment (SELEX). The method is further explained by the development of nucleic acid ligands which show thermal dependent binding to DNA polymerases. WO-A-96/41010 reports about two method to minimize side reactions of the so-called polymerase chain reaction. In the first method termed Hotstart PCR all of the reagents are heated to 72EC before adding the final reagents, usually the polymerase. Hot start PCR has certain drawbacks; the requirement of reopening of tubes before initiating thermocycling increases crossover contamination and is tedious when handling a large number of samples. In the second method a neutralizing antibody to Taq-polymerase (referred to its TaqStart) is added to the complete reaction mixture. This antibody inhibits the polymerase activity at ambient temperature (Kellogg et al. 1994, BioTechniques 16: 1134 - 1137). The antibody is inactivated by heat denaturation once the reaction is thermocycled, rendering the polymerase active. The drawback of this approach to reduce side products is that the anti-taq antibody should be stored at - 20°C until use which means that detection kits should packaged and shipped under controlled environment adding to the cost. Furthermore, a significant amount of antibody is needed.

WO-96/41010 addresses the problem caused by the drawbacks of the Hotstart or the TaqStart method and provides several oligonucleotides being capable to inhibit a polymerase at ambient temperature but loosing there inhibiting properties with higher temperatures, especially those temperatures where the PCR reaction is working.

Using the proposed oligonucleotides there are several disadvantages: First of all this concerns the incomplete loss of inhibition properties of the oligonucleotide inhibitors after heating the reaction mixture above the proposed polymerase activation temperature and the reversibility of the inhibition properties of the oligonucleotide inhibitors of Taq polymerase after cooling down the reaction temperature. Other disadvantages concern the high costs of the proposed oligonucleotide inhibitors due to the length of oligonucleotide sequences and the amount of oligonucleotide needed per reaction.

The present invention provides oligonucleotide inhibitor of polymerase activity of thermostable polymerases, the oligonucleotide having a first structural domain (stem I), a second structural domain (loop), and a third structural domain (stem II), wherein
the third structural domain (stem II) has a nucleotide sequence which is complementary to the oligonucleotide sequence of the first structural domain (stem I), the first and third structural domain are forming a first double stranded structural element (stem) by hybridization of the complementary oligonucleotide sequences at ambient temperature,
the second structural domain (loop) comprising a structural element NN'XY, wherein
   - N and N' is a nucleotide residue of the pyrimidine type or
   - N is a nucleotide residue of the purine type and N' is a nucleotide residue of the pyrimidine type,
   - X is a structural element of nucleotide residues within the loop formed by a turn portion in the loop and a double stranded portion, wherein the double stranded portion is consisting of two to five nucleotide residues of any kind, and
   - the turn portion is consisting of one to four nucleotide residues of any kind,
   - Y is a structural element consisting of up to 4 nucleotide residues of any kind.

The oligonucleotides of the invention are advantageous since
- The irreversibility of the loss of inhibitor activity of the oligonucleotide inhibitor
- The possibility of shortening the original oligonucleotide sequence, what results in decreasing the cost of oligonucleotide synthesis,
- The improved activity of the oligonucleotide inhibitor what opens the possibility of decreasing the needed concentration of the used oligonucleotide inhibitor,
- The possibility of determining the temperature point or the temperature range where the inhibitor activity gets lost by varying the oligonucleotide secondary structure.

The estimated irreversibility of the loss of inhibitor activity of the oligonucleotide of the present invention against the polymerase activity of thermostable DNA polymerases as well as the loss of its inhibitor activity by itself seems to be due to the 5'-3'-nucleolytic activity of the DNA polymerase. The oligonucleotide inhibitor will be cleaved by the thermostable polymerase at a discrete sequence point, which strongly depends on the secondary structure. In a different embodiment the polymerase inhibitor of the present invention can be used in order to inhibit the DNA polymerase in a reversible fashion by using a thermostable DNA polymerase with altered 5'-3'-nucleolytic activity.

Preferably, the oligonucleotide inhibitor of the present invention comprises the stem which is formed by the first and third structural domain consists of at least 14 base pairs. In another preferred embodiment the stem formed by the first and third structural domain consists up to 20 base pairs.

The thermostable polymerase is in particular a DNA polymerase which derived from bacteria of the genus Thermus.

The oligonucleotides according to the invention can be present in PCR kits which contains at least the thermostable DNA polymerase and the oligonucleotide inhibitor either in separated containers or in a premixed form, whereby the latter variant will be preferably used for thermostable DNA polymerases with altered 5'-3'-nucleolytic activity.

### Example 1

### Hot-Start Oligonucleotides (HSO) effect determination

To show the Hot Start effect on different oligonucleotides we used hairpin extension assay. 5'-[³²P]-labelled 49-mer oligonucleotide which forms a hairpin-like structure due to the formation of a stem-loop structure with four thymidines in the loop was chosen as a model DNA template in the extension experiments in the presence of different Hot-Start Oligonucleotides.
[³²P]-ATG-CCT-AAG-TTT-CGA-ACG-CGG-CTA-GCC-AGC-TTT-TGC-TGG-CTA-GCC-GCG-T

Two reaction mixtures were prepared:

| Mix 1: | |
|---|---|
| [³²P]-labelled oligonucleotide | (15,000 cpm) |
| Non-labelled oligonucleotide | 1 pmol |
| H₂O up to a total volume per reaction of | 15 µl |

| Mix 2: | |
|---|---|
| TaqPol | 1 unit |
| ΣdNTP | 1.25 nmol each |
| Hot-Start Oligonucleotide | 1pmol |
| 10x TaqPol buffer | 1 µl |
| H₂O up to a total volume per reaction of | 10 µl |

Steps of the experiment:
1. Mix 2 was held out at room temperature for 30 minutes.
2. Mix 1 was added.
3. The combined mixture was incubated at four different temperatures: 30°C, 35°C, 40°C and 45°C for one hour.
4. The reaction was stopped by addition of 1 µl EDTA (0.5 mol/l).
5. The reaction mixture was evaporated, dissolved in 80% formamide, denatured at 95°C for five minutes, put on ice for five minutes and loaded on the 20 % polyacylamide gel.

The Hot-Start effect was determined by autoradiography of the gel. From the data obtained the following could be concluded.

Dependent on the type of the Hot-Start Oligonucleotide (HSO) in the mixture the extension of the model DNA template was different and also dependent on the incubation temperature. For example, in case of the HSO having Seq. ID. No. 2 Q21-13 the extension was completely inhibited at 30°C and 35°C, partially inhibited at 40°C and no inhibition was observed at 45°C.

All oligonucleotides were synthesized using standard phosphoamidite solid-phased synthesis scheme in ASM 102-U DNA synthesizer (Bioset, Russia). The oligonucleotide was labelled by T4-polynucleotide kinase (MBI, Fermentas) with ³²γ-ATP (Obnynsk, Russia).

### Example 2:

To show the efficiency of the HSO's two PCR mixutres are prepared:

| PCR-Mixture 1 - without HSO: | |
|---|---|
| 10x PCR-Buffer (0.1% Tween, 660 mM TrisHCl pH 8.8, 166 mM (NH₄)₂SO₄) | 5.0 µl |
| MgCl₂(25mM) | 5.0 µl |
| dNTPs (2mM each) | 5.0 µl |
| α-[³²P]-dCTP (110 TBq/mmol) | 1 µl |
| DNA template (0.2 ng/µl) | 5.0 µl |
| primer A (30 pmol) | 1.0 µl |
| primer B (30 pmol) | 1.0 µl |
| H₂O | 26.8 µl |

| PCR-Mixture II - with HSO: | |
|---|---|
| 10X PCR-Buffer (0.1% Tween, 660 mM Tris-HCl pH 8.8, 166 mM (NH₄)₂SO₄) | 5.0 µl |
| MgCl₂(25mM) | 5.0 µl |
| dNTPs (2mM each) | 5.0 µl |
| α-[³²P]-dCTP (110 TBq/mmol) | 1 µl |
| primer A (30 pmol) | 1.0 µl |
| primer B (30 pmol) | 1.0 µl |
| H₂O | 24.8 µl |

The target-DNA is amplified in a thermocycler (Perkin. Elmer GeneAmp PCR System 9700) with heatable lid. In the beginning the target-DNA is denaturated at 95°C for 5 min and cooled to 20°C.

To Mixture I and II DNA polymerase (5 U/µl, 0.2 µl) is added, however, the Hot-Start Oligo (0.5 pmol/µl, 2.0 µl) is added only to Mixture II.

After addition both mixtures are incubated at 35°C for 30 sec. This is to be regarded as a first PCR cyclus. The analysis by gel electrophoresis and counting of the radioactive decay shows that only Mixture I contains radioactive labelled double-stand DNA, whereas the HSO in Mixture II inhibited the polymerase to extend the primers by incorporation of any dNTP at this non-optimal temperature.

The PCR conditions are:

| | | |
|---|---|---|
| 30 sec. | 94°C | denaturation |
| 15 sec. | 56°C | primer annealing |
| 20 sec. | 72°C | extension |

To finish the PCR programme a further elongation for 1 min at 72°C is added before cooling to 4°C.

## Claims

1. An oligonucleotide inhibitor of the polymerase activity of thermostable polymerases, the oligonucleotide having a first structural domain (stem I), a second structural domain (loop), and a third structural domain (stem II), wherein
the third structural domain (stem II) is consisting of oligonucleotides having a nucleotide sequence which is complementary to the oligonucleotide sequence of the first structural domain (stem I), the first and third structural domain are forming a first double stranded structural element (stem) by hybridization of the complementary oligonucleotide sequences at ambient temperature,
the second structural domain (loop) comprising a structural element NN'XY, wherein
- N and N' is a nucleotide residue of the pyrimidine type or
- N is a nucleotide residue of the purine type and N' is a nucleotide residue of the pyrimidine type,
- X is a structural element of nucleotide residues within the loop formed by a turn portion in the loop and a double stranded portion, wherein the double stranded portion is consisting of two to five nucleotide residues of any kind, and
- the turn portion is consisting of one to four nucleotide residues of any kind,
- Y is a structural element consisting of up to 4 nucleotide residues of any kind.

2. The oligonucleotide according to claim 1, wherein the stem formed by the first and third structural domain consists of at least 14 base pairs.

3. The oligonucleotide according to claim 2, wherein the stem formed by the first and third structural domain consists of up to 20 base pairs.

4. The oligonucleotide according to claims 1 to 3, wherein the thermostable polymerase is selected from the group consisting of DNA polymerases from bacteria of the genus Thermus.

5. The oligonucleotide according to claim 4, wherein the thermostable DNA polymerase has an altered 5'-3'-nucleolytic activity compared with its natural 5'-3'-nucleolytic activity.

6. The oligonucleotide according to claim 1 to 5 having the Seq. ID No. 1 to 12.

7. A method for inhibition of the polymerase activity of thermostable polmyerases by providing an inhibitor according to any of the claims 1 to 6 to a sample containing nucleic acid to be amplified.

8. A PCR kit, which contains at least the thermostable DNA polymerase and in a separated container the oligonucleotide inhibitor of the respective polymerase.

9. A PCR kit, which contains at least the thermostable DNA polymerase and the oligonucleotide inhibitor of the respective polymerase in a premixed form.

10. A PCR kit according to claim 7 and 8, wherein the DNA polymerase has an altered 5'-3'-nucleolytic activity compared with its natural 5'-3'-nucleolytic activity.


SEQUENCE LISTING

(1) GENERAL INFORMATION:

(i) APPLICANT:
(A) NAME: MIRA Diagnostica GmbH
(B) STREET: Hemmelrather Weg 201
(C) CITY: Leverkusen
(E) COUNTRY: Germany
(F) POSTAL CODE (ZIP): 51377

(ii) TITLE OF INVENTION: An oligonucleotide inhibitor of thermostable
polymerase

(iii) NUMBER OF SEQUENCES: 17

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 62 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 1:

GGCGGAGCGA TCATCTCAGA GCATTCTTAG CGTTTTGTTC TTGTGTATGA TTCGCTTTTC 60
CC 62

(2) INFORMATION FOR SEQ ID NO: 2:

(i) SEQUENCE CHARACTERISTICS:
(A) LENGTH: 46 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 2:

GCGGTCGGCT CGGGGCATTC TTAGCGTTTT GCCCCGAGCC GACCGC 46

(2) INFORMATION FOR SEQ ID NO: 3:

(i) SEQUENCE CHARACTERISTICS:
(A) LENGTH: 78 base pairs
(B) TYPE: nucleic acid
(C) STRANDEDNESS: unknown

(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 3:

TTCTCGGTTG GTCTCTGGCG GAGCGATCAT CTCAGAGCAT TCTTAGCGTT TTGTTCTTGT 60
GTATGATTCG CTTTTCCC 78

(2) INFORMATION FOR SEQ ID NO: 4:

(i) SEQUENCE CHARACTERISTICS:
(A) LENGTH: 49 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 4:

GCGATCATCT CAGAGCATTC TTAGCGTTTT GTTCTTGTGT ATGATTCGC 49

(2) INFORMATION FOR SEQ ID NO: 5:

(i) SEQUENCE CHARACTERISTICS:
(A) LENGTH: 40 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 5:

GCGGCTCAGA GCATTCTTAG CGTTTTGTTC TTGTGTCGGC 40

(2) INFORMATION FOR SEQ ID NO: 6:

(i) SEQUENCE CHARACTERISTICS:
(A) LENGTH: 48 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 6:

GCGATCATCT CAGAGCATTC GCTGCGCGTT TTGTTCTGAG ATGATCGC 48

(2) INFORMATION FOR SEQ ID NO: 7:

(i) SEQUENCE CHARACTERISTICS:
(A) LENGTH: 36 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 7:

CAAGACCAGA CAATGTACAG TATTGGCCTG ATCTTG 36

(2) INFORMATION FOR SEQ ID NO: 8:

(i) SEQUENCE CHARACTERISTICS:
(A) LENGTH: 49 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 8:

GATCGAAAGC GATCATCTCA GAGCATTCTT AGCGTTTTGC TCTGAGATT 49

(2) INFORMATION FOR SEQ ID NO: 9:

(i) SEQUENCE CHARACTERISTICS:
(A) LENGTH: 46 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 9:

GCGATCATCT CAGAGCATTC TTAGCGTTTT GTTCTGAGAT GATCGC 46

(2) INFORMATION FOR SEQ ID NO: 10:

(i) SEQUENCE CHARACTERISTICS:
(A) LENGTH: 44 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 10:

GGAGCAAGAC CAGACAATGT ACAGTATTGG CCTGATCTTG CTCC 44

(2) INFORMATION FOR SEQ ID NO: 11:

(i) SEQUENCE CHARACTERISTICS:
(A) LENGTH: 44 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 11:

GGAGCAAAGA CCAGCCAATG TACAGTATTG GCTGGTCTTG CTCC 44

(2) INFORMATION FOR SEQ ID NO: 12:

(i) SEQUENCE CHARACTERISTICS:
(A) LENGTH: 46 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 12:

GCGATCATCT CAGAGCATTC GTCGCGTTTT GTTCTGAGAT GATCGC 46

(2) INFORMATION FOR SEQ ID NO: 13:

(i) SEQUENCE CHARACTERISTICS:
(A) LENGTH: 26 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 13:

CAGAGCATTC TTAGCGTTTT GTTCTG 26

(2) INFORMATION FOR SEQ ID NO: 14:

(i) SEQUENCE CHARACTERISTICS:
(A) LENGTH: 22 base pairs
(B) TYPE: nucleic acid
(C) STRANDEDNESS: unknown
(D) TOPOLOGY: unknown

(ii) MOLECULE TYPE: other nucleic acid

(A) DESCRIPTION: /desc = "DNA-Oligonucleotides"

(xi) SEQUENCE DESCRIPTION: SEQ ID NO: 14:

GGGCGTTCTT AGCGTTTCGC CC 22

(2) INFORMATION FOR SEQ ID NO: 15:

(i) SEQUENCE CHARACTERISTICS:
(A) LENGTH: 17 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 15:

GCGGTGTACA GTACCGC 17

(2) INFORMATION FOR SEQ ID NO: 16:

(i) SEQUENCE CHARACTERISTICS:
(A) LENGTH: 46 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 16:

GCGATCATCT CAGAGCATTC TTTTCGTTTT GTTCTGAGAT GATCGC 46

(2) INFORMATION FOR SEQ ID NO: 17:

(i) SEQUENCE CHARACTERISTICS:
(A) LENGTH: 49 base pairs
(B) TYPE: nucleic acid
(C) STRANDEDNESS: unknown
(D) TOPOLOGY: unknown

(ii) MOLECULE TYPE: other nucleic acid
(A) DESCRIPTION: /desc = "DNA-Oligonucleotides"

(xi) SEQUENCE DESCRIPTION: SEQ ID NO: 17:

ATGCCTAAGT TTCGAACGCG GCTAGCCAGC TTTTGCTGGC TAGCCGCGT 49